# EUROPÄISCHE PATENTSCHRIFT

(11) **EP 0 658 346 B1**
(45) Veröffentlichungstag und Bekanntmachung des Hinweises auf die Patenterteilung: **08.12.1999**
(21) Anmeldenummer: 94115440.3
(22) Anmeldetag: 30.09.1994
(51) Int. Cl.: A61K 38/46

(54) **Verwendung von hydrolytischen Enzymen zur Abortprophylaxe bei Schwangeren mit idiopathischem Abortus habitualis in der Anamnese**
Use of hydrolytic enzymes for prophylaxis of abortion at pregnant women with idiopathic abortus habitualis in the anamnesis
Utilisation des enzymes hydrolytiques pour la prophylaxie d'avortement aux femmes enceintes avec abortus habitualis protopathique dans l'anamnèse

(30) Priorität: 25.10.1993 DE 4336343
(43) Veröffentlichungstag der Anmeldung: 21.06.1995
(73) Patentinhaber: MUCOS Pharma GmbH & Co., D-82538 Geretsried (DE)
(72) Erfinder: Ransberger, Karl, D-82402 Seeshaupt (DE); Dittmar, Friedrich-Wilhelm, Prof. Dr., D-82319 Söcking (DE); Kunze, Rudolf, Dr., D-12353 Berlin (DE); Stauder, Gerhard, Dr., D-82515 Wolfratshausen (DE)
(74) Vertreter: Grünecker, Kinkeldey, Stockmair & Schwanhäusser Anwaltssozietät

(56) Entgegenhaltungen:
- BEITR|GE ZUR INFUSIONS- THERAPIE, Band 3: Trans- fusionsmedizin 1989/90 G. MUELLER-ECKHARDT et al. "Passive Immuntherapie mit polyvalenten Immunglobulinen bei Frauen mit habituellen Aborten" KARGER-Verlag, M nchen 1990 Seiten 298-301

## Beschreibung

Die vorliegende Erfindung betrifft die Verwendung von mindestens einem hydrolytischen Enzym Zur Herstellung eines Medikaments zur Abortprophylaxe bei Schwangeren mit idiopathischem Abortus habitualis in der Anamnese.

Ein habitueller Abort liegt vor, wenn mindestens drei aufeinanderfolgende Schwangerschaften vor der 28. Schwangerschaftswoche mit einem spontanen Abort enden (Pschyrembel, Klinisches Wörterbuch, 255. Auflage, Walter de Gruyter, 1986). Habituelle spontane Aborte lassen sich in manchen Fällen auf anatomische, genetische oder hormonelle Anomalitäten der betreffenden patientinnen zurückführen. Beispiele hierfür sind congenitale Uterusanomalien, Chromosomenanomalien, Schilddrüsenstörungen, Diabetes mellitus, Autoimmunkrankheiten, virale oder bakterielle Infektionen u.a. (Makino, T. et al., Annals New York Academy of Sciences, Seiten 597 - 604, 1990). Mit einer Inzidenz von 0,4 % tritt diese Art des Aborts jedoch idiopathisch, d.h. ohne erkennbare Ursachen (Dispositionen) auf.

Therapeutische bzw. prophylaktische Maßnahmen, die bisher bei drohendem Abort ohne Hinweis auf eine mögliche Ursache zur Erhaltung der Schwangerschaft durchgeführt werden, sind Cervicalcerclage, Hormonsupplementierung (Östrogen, Gestagen, Progesteron, etc.) sowie die Verabreichung allogener Leukozyten und polyvalenter Immunglobuline.

Die Prophylaxe von habituellem Abort mit Kombinationspräparaten auf Hormonbasis führt erfahrungsgemäß nur bei jeder zehnten Schwangeren zu einer regelrechten Geburt.

Höhere Erfolgsraten in Fällen eines habituellen Aborts werden durch sog. Immuntherapien erzielt. Die Mechanismen der Immunregulation in der normalen Schwangerschaft sind weitgehend ungeklärt. Jedoch wurde gefunden, daß eine passive Immuntherapie bei Schwangeren mit Abortrisiko in 80 % der Fälle erfolgreich ist. Dabei werden den betroffenen Frauen mehrfach polyvalente Immunglobuline bis zur ca. 25. Schwangerschaftswoche intravenös verabreicht. Die Infusionen wurden in der Regel gut vertragen, wobei leichte Nebenwirkungen, wie Kopfschmerzen, Schwindelgefühle oder Temperaturerhöhung bei der jeweils ersten Infusion nicht auszuschließen sind (Mueller-Eckhardt, G. et al., Beitr Infusionsther. Basel, Karger 26, Seiten 298 - 301, 1990). Die Kosten dieser Therapie, bei der bis zu ca. 150 g Immunglobulin verabreicht werden, sind allerdings beachtlich.

Eine weitere Form der Immuntherapie ist die meist zweimalige Behandlung mit allogenen Leukozyten. Die publizierten Erfolgsraten liegen zwischen 30 und 80 % und scheinen unabhängig davon zu sein, ob Zellen des Partners oder von Drittspendern isoliert werden. Die Nebenwirkungen sind den Berichten nach gering. Es ist allerdings hervorzuheben, daß eine Leukozytentherapie fast immer eine HLA-Immunisierung zur Folge hat, die für die behandelten Frauen im Falle einer späteren medizinisch notwendigen Organ- und/oder Bluttransfusion von erheblichem Nachteil sein kann (Clark, D.A. and Daya, S., Am. J. Reprod. Immunol. 25, Seiten 18 - 24, 1991).

Aufgabe der vorliegenden Erfindung ist die Bereitstellung eines kostengünstigen und gut verträglichen Arzneimittels, mit dem Frauen mit idiopathischem, habituellem Abort behandelt werden können, um eine normale Schwangerschaft zu ermöglichen.

Diese Aufgabe wird erfindungsgemäß dadurch gelöst, daß mindestens ein hydrolytisches Enzym zur Herstellung eines Medikaments zur Behandlung der vorgenannten Indikation verwendet wird.

Es wurde überraschenderweise gefunden, daß hydrolytische Enzyme, die bisher vor allem zur Langzeitbehandlung bei Tumoren, Zusatzbehandlung während der Strahlentherapie, Unterstützung bei Entzündungen und Virusinfektionen, rheumatischen Erkrankungen und Durchblutungsstörungen verwendet wurden, zur Verhinderung einer weiteren Fehlgeburt bei Frauen mit idiopathischem, habituellem Abort eingesetzt werden können.

Als hydrolytische Enzyme können erfindungsgemäß sowohl pflanzliche als auch tierische Proteasen sowie eine Kombination von pflanzlichen und tierischen Proteasen, verwendet werden. Die pflanzlichen Proteasen Bromelain und Papain und tierischen Proteasen Trypsin und Chymotrypsin haben sich erfindungsgemäß als besonders wirkungsvoll erwiesen.

Bromelain ist nach üblichen Verfahren aus dem Preßsaft der Ananas isolierbar.

Papain ist ein proteolytisches Enzym, das aus dem Milchsaft der unreifen, fleischigen Früchte des Melonenbaums Carica papaya durch übliche Verfahren herstellbar ist.

Trypsin und Chymotrypsin sind proteolytische Enzyme, die in an sich bekannter Weise aus Pankreas gewonnen werden können.

Weiterhin kann vorzugsweise zusätzlich Rutin (internationaler Freiname: Rutosid), ein zu den Flavonoiden gehörendes Glukosid verwendet werden.

Besonders bevorzugt ist die erfindungsgemäße Verwendung von hydrolytischen Enzymen in Kombination mit Gestagenen und/oder Östrogenen zur Unterstützung der Schwangerschaft. Dabei eignen sich vor allem Hydroxyprogesteroncaproat als Gestagen und Estradiolvalerat als Östrogen.

Für die Herstellung von pharmazeutischen Präparaten, die die erfindungsgemäßen Enzyme enthalten, können weiterhin übliche Hilfs- und/oder Trägerstoffe verwendet werden.

Eine besonders gute Wirksamkeit hat die kombinierte Verwendung von 20 bis 100 mg Bromelain, 40 bis 120 mg Papain, 10 bis 60 mg Trypsin, 20 bis 60 mg Chymotrypsin und 50 bis 150 mg Rutosid x 3 H₂O pro Dosiseinheit.

Vorzugsweise werden folgende Mengen verwendet:
a) 100 mg Papain, 40 mg Trypsin und 40 mg Chymotrypsin;
b) 90 mg Bromelain, 48 mg Trypsin und 100 mg Rutosid x 3 H₂O; oder
c) 45 mg Bromelain, 60 mg Papain und 24 mg Trypsin.

Das folgende klinische Beispiel erläutert die Erfindung.

### Klinisches Beispiel

12 schwangeren Frauen mit der Indikation eines idiopathischen, habituellen Aborts wurden die hydrolytischen Enzyme Papain, Trypsin und Chymotrypsin oral verabreicht. Die Behandlung mit zwei Enzymtabletten (Wobe-Mucos® mit einem Gehalt von jeweils 100 mg Papain, 40 mg Trypsin und 40 mg Chymotrypsin, dreimal pro Tag) wurde vor der 12. Schwangerschaftswoche begonnen. Zusätzlich wurden Östrogen/Gestagen (Gravibinon® mit einem Gehalt von 250 mg Hydroxyprogesteroncaproat und 5 mg Estradiolvalerat in 1 ml Injektionslösung, Anfangsdosis 2 ml/Tag, Behandlungsdosis 1 ml/alle 2 Tage) verabreicht.

In allen Fällen überdauerte die Schwangerschaft die 28. Woche. Der Apgar-Index der Neugeborenen betrug 9 bis 10; das Geburtsgewicht der Neugeborenen und Placentagewicht lagen innerhalb der physiologischen Grenzen.

Die Enzymtherapie hatte eine hohe Verträglichkeit. Nebenwirkungen wurden nicht festgestellt. Das klinische Beispiel zeigt, daß hydrolytische Enzyme überraschenderweise erfolgreich zur Verhinderung eines Aborts verwendet werden können.

## Patentansprüche

1. Verwendung von mindestens einem hydrolytischen Enzym zur Herstellung eines Medikaments zur Abortprophylaxe bei Schwangeren mit idiopathischem Abortus habitualis in der Anamnese.

2. Verwendung nach Anspruch 1, **dadurch gekennzeichnet**, daß als hydrolytisches Enzym eine pflanzliche Protease verwendet wird.

3. Verwendung nach Anspruch 2, **dadurch gekennzeichnet**, daß Bromelain und/oder Papain verwendet wird.

4. Verwendung nach Anspruch 1, **dadurch gekennzeichnet**, daß als hydrolytisches Enzym eine tierische Protease verwendet wird.

5. Verwendung nach Anspruch 4, **dadurch gekennzeichnet**, daß Trypsin und/oder Chymotrypsin verwendet wird.

6. Verwendung nach einem der Ansprüche 1 bis 5, **dadurch gekennzeichnet**, daß zusätzlich Rutin verwendet wird.

7. Verwendung nach einem der Ansprüche 1 bis 6, **dadurch gekennzeichnet**, daß zusätzlich mindestens ein Gestagen und/oder mindestens ein Östrogen verwendet werden.

8. Verwendung nach Anspruch 7, **dadurch gekennzeichnet**, daß als Gestagen Hydroxyprogesteroncaproat und als Östrogen Estradiolvalerat verwendet werden.

## Claims

1. Use of at least one hydrolytic enzyme for producing a drug for the prophylaxis of abortion in pregnant women with habitual idiopathic abortion in their anamneses.

2. Use according to claim 1, **characterized in** that a vegetable protease is used as said hydrolytic enzyme.

3. Use according to claim 2, **characterized in** that bromelain and/or papain are used.

4. Use according to claim 1, **characterized in** that an animal protease is used as said hydrolytic enzyme.

5. Use according to claim 4, **characterized in** that trypsin and/or chymotrypsin are used.

6. Use according to any one of claims 1 to 5, **characterized in** that rutin is additionally used.

7. Use according to any one of claims 1 to 6, **characterized in** that at least one gestagen and/or at least one estrogen are additionally used.

8. Use according to claim 7, **characterized in** that hydroxyprogesterone caproate is used as gestagen, and estradiol valerate as estrogen.

## Revendications

1. Utilisation d'au moins une enzyme hydrolytique pour préparer un médicament destiné à la prophylaxie de l'avortement chez les femmes enceintes présentant une anamnèse d'avortement habituel idiopathique.

2. Utilisation selon la revendication 1, caractérisée en ce qu'on utilise en tant qu'enzyme hydrolytique une protéase végétale.

3. Utilisation selon la revendication 2, caractérisée en ce qu'on utilise de la bromélaïne et/ou de la papaïne.

4. Utilisation selon la revendication 1, caractérisée en ce que l'on utilise en tant qu'enzyme hydrolytique une protéase animale.

5. Utilisation selon la revendication 4, caractérisée en ce qu'on utilise de la trypsine et/ou de la chymotrypsine.

6. Utilisation selon l'une des revendications 1 à 5, caractérisée en ce qu'on utilise en outre de la rutine.

7. Utilisation selon l'une des revendications 1 à 6, caractérisée en ce qu'on utilise en outre au moins un gestagène et/ou au moins un oestrogène.

8. Utilisation selon la revendication 7, caractérisée en ce qu'on utilise du caproate d'hydroxyprogestérone en tant que gestagène et du valérate d'oestradiol en tant qu'oestrogène.
